# EUROPEAN PATENT APPLICATION

(11) **EP 1 955 664 A1**
(43) Date of publication of application: **13.08.2008**
(21) Application number: 07101777.6
(22) Date of filing: 06.02.2007
(51) Int. Cl.: A61B 17/11, A61B 17/00

(54) **Device for bypass surgery**

(71) Applicant: Universiteit Utrecht Holding B.V., 3584 CM Utrecht (NL)
(72) Inventor: Verkerk, Jacob, 1053 LM Amsterdam (NL); Jansen, Erik Willem Lodewijk, 3707 ES Zeist (NL)
(74) Representative: van Loon, C.J.J.

(57) **Abstract**

An apparatus for bypass surgery on body channels such as blood vessels, comprising a body with at least a coupling element for coupling to a first body channel and a flange part with at least one cut, wherein at least a first part of the flange part which adjoins the cut is at least partly bent and/or displaced with respect to a part of the flange part on an opposite part of the cut.

## Description

The invention relates to an apparatus for interconnecting at least two body channels.

Body channels in human or animal bodies are of vital importance for many organic processes. For instance, blood vessels such as arteries, veins and vessels ensure good blood circulation in the body, supply and discharge of auxiliary substances and waste substances, supply of oxygen to organs and the like. If such a body channel becomes wholly or partly blocked or breaks down otherwise, it may be necessary to bypass or replace this affected part of the body channel. To this end, a replacement vessel may be used which is, in flow direction of the body channel, connected before and after the respective affected part or is connected to the respective body channel before the affected part and connected with another body channel. Such surgery is generally referred to as bypass surgery.

In practice, making a bypass is realized by connecting two vessels using needle and thread (anastomosis). To this end, in the wall of the blood vessel to be connected, an opening needs to be provided before the bypass blood vessel can be connected, which means that the flow of blood through the respective blood vessel to be connected needs to be stopped. In, for instance, cardiac surgery, this may mean that the blood circulation temporarily needs to be taken over by a heart-lung machine, which is particularly taxing for the patient. In addition, such a method requires the surgeon to have special skills. Automation of a vessel coupling can further contribute to a simplification of the operation.

From WO 2005/060836, an apparatus is known for bypass surgery, where a ring-shaped element wound from wire with at least more than one winding and at least one sharp end is provided. The sharp end is run through the wall of a vessel to be connected, after which the ring-shaped element is rotated, such that at least one winding is introduced into the vessel and at least a part of a further winding is kept outside the vessel, such that the wall of the vessel is clamped between the two windings and is fixed with glue. By an open end, the bypass vessel is fixed to the winding located outside the vessel with the aid of needle and thread or a clamping mechanism. After this, a head of a laser is inserted into the bypass vessel, with which the part of the wall of the vessel clamped inside the windings is cut loose and suctioned out, for making a connection. Upon removing the laser, the bypass is temporarily clamped shut in order to prevent blood flowing away through that vessel before the opposite end is fixed to the same or another blood vessel in a similar manner.

This method has the advantage that the ring used provides a good surface for fixing the bypass vessel, so that it also enables microsurgery, for instance on brains. In most cases, the native blood circulation does not need to be stopped during surgery. A drawback of this technique is that a laser needs to be used therein, which is relatively complex.

The invention contemplates an apparatus for bypass surgery.

In a first aspect, an apparatus according to the invention is characterized by a body with at least a coupling element for coupling to a first body channel and a flange part with at least one cut, while at least a first part of the flange part which adjoins the cut is at least partly bent and/or displaced with respect to a part of the flange part on an opposite part of the cut.

The displaced part offers the possibility to run above-mentioned edge at least partly through a cut in a wall of a body channel, after which the flange part can be brought under the respective wall by rotation, while above-mentioned wall at least partly elastically deforms. The displacement is preferably such that, in side elevational view, an edge of above-mentioned first part proximal to the cut is located at a distance from an edge of the opposite part proximal to the cut, while, in bottom view of the apparatus, the two edges are virtually next to one another. A bypass vessel can then be coupled with the coupling element or be already connected thereto.

In a second aspect, the invention is characterized in that the coupling element is tubular, while the flange part extends from near an end thereof, in radial direction. Here, during use, the coupling element can reach through a cut in a wall of a vessel, while the flange part abuts the inside of above-mentioned wall and the wall has at least partly been slightly elastically deformed and abuts the coupling element and/or the flange part. Preferably, a sealing is directly obtained therewith.

In a third aspect, the invention is characterized by a set according to claim 15.

In a further aspect, the invention is characterized by a method according to claim 19 or a method for bypass surgery according to claim 23.

With use of an apparatus or method according to the invention, the apparatus can simply be provided by making a small incision in a wall of a body channel such as a blood vessel, for instance with a scalpel, or using the apparatus itself, after which the apparatus can be inserted, without a part of the wall needing to be removed.

The invention will be explained in more detail with reference to the drawing, in which:
Fig. 1 shows, in perspective view, an apparatus according to the invention;
Figs. 2A and B show, in side elevational view and top plan view, an apparatus according to Figure 1;
Figs. 3A - E show, in five steps, the placement of an apparatus in the wall of a vessel;
Fig. 4 shows, in cross-sectional side view, an apparatus according to the invention placed in a blood vessel;
Fig. 5 schematically shows a connection between two blood vessels, provided with an apparatus and method according to the invention; and
Figs. 6A and B show, in side elevational view and partly transparently shown top plan view, respectively, an alternative connection according to the invention.

The embodiments shown and described of the invention only serve as an illustration and should not be taken as being limitative in any way. Many variations thereof are possible within the framework of the invention set forth in the claims.

In this description, as a body channel, always a blood vessel will be discussed such as an artery, vein or vessel, which can be located in any place in a human or animal body, or, particularly for training purposes, outside it. However, a similar apparatus or method may be used in or for different body channels such as air channels, bowels and the like. Herein, a body channel and in particular a bypass channel or bypass vessel may be an autograft, allograft or xenograft, or an artificial vessel, combined or not combined and provided or not provided with additional elements such as for instance a stent.

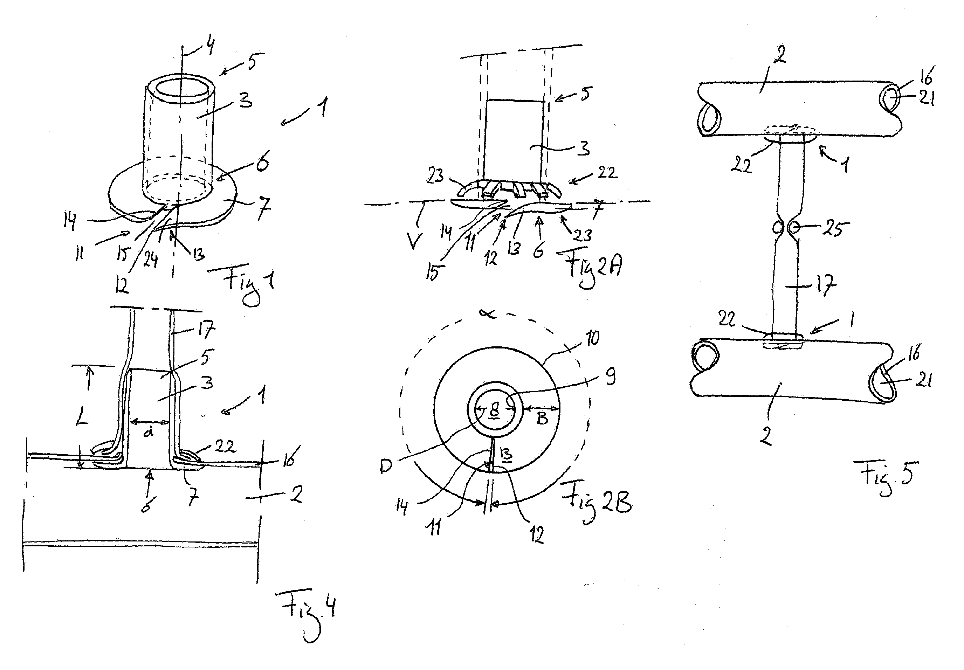

Fig. 1 shows an apparatus 1 for coupling to a wall of a blood vessel 2. This apparatus 1 comprises a tubular coupling element 3 with a longitudinal axis 4. In the embodiment shown, the tubular coupling element 3 has a substantially circular cross section, at right angles to the longitudinal axis 4. The coupling element has a first end 5 and an opposite second end 6, from which a substantially ring-shaped flange part 7 extends outwards, through an angle α of more than 320 degrees, in particular about 360 degrees around the longitudinal axis, viewed in top plan view (Fig. 2B). The flange part 7 has a central opening 8 of which a longitudinal edge 9 is determined by the second end 6 of the coupling element 3, and an outer longitudinal edge 10. The longitudinal edges 9, 10 are preferably placed concentrically around the longitudinal axis 4. In the drawing, for clarity, the length L of tube part 3 is drawn relatively large. Preferably, it has a length L between the first and second end 5, 6 which is approximately equal to between 0.5 and 3 times the diameter D of the tube part so that, on the one hand, a smallest possible contact surface between the coupling element and blood is obtained and, on the other hand, sufficient stability of the connection. The width B of the flange part 7, in radial direction, is, for instance, between 0.1 and 3 times a diameter D of the central opening 8, more in particular between 0.5 and 1.5 times. In the exemplary embodiment shown, the width B is approximately equal to the radius ½ D.

As is clearly visible in Figs. 2A and B, in the flange part 7, a cut 11 is provided, which, in the example shown, extends approximately radially from the coupling element 3 to the outer longitudinal edge 10. A cut 11 may be provided physically in a completely ring-shaped flange part 7, but may also be provided directly during the manufacture of the flange part 7, so that the flange part 7 is not completely ring-shaped during production. The cut 11 is bounded by at least a longitudinal edge 12 of a first part 13 of the flange part 7 and a second longitudinal edge 14 of an opposite end of the flange part 7. The first part 13 is bent slightly downwards, that is to say, in a direction away from the coupling element 3, such that the first longitudinal edge is located at least partly below an imaginary plane V determined by the first longitudinal edge, viewed in side elevational view below the second longitudinal edge 14 (viewed in the longitudinal direction of the longitudinal axis 4 at a distance therefrom), so that, between the first and second longitudinal edge 12, 14, a slit 15 is obtained. The flange part 7 may be cut loose somewhat from the coupling element 3 at the height of the cut 11, for instance across the length of the first part 13, so that the first and second longitudinal edge 12, 14 extend approximately parallel to each other. However, the first longitudinal edge 12 may also extend in an inclined manner with respect to the longitudinal axis 4.

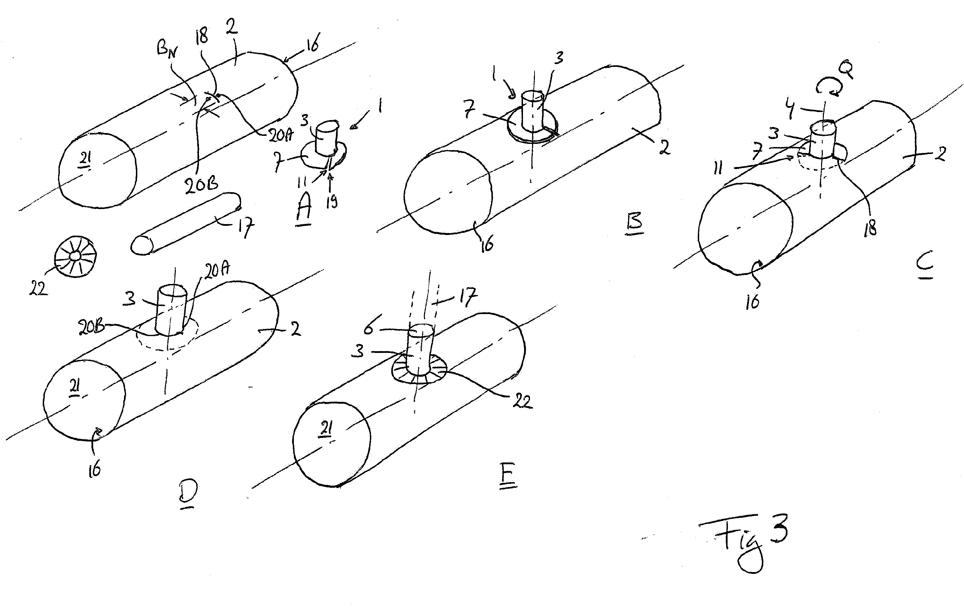

Fig. 3A shows a wall 16 of a vessel (xenograft or autograft) 2 to which a bypass vessel 17 is to be connected. Apparatus 1 and bypass vessel 17 together form a set or at least a part thereof and may be both intercouplable, for instance during surgery, and already be interconnected, for instance integrally manufactured or connected by, for instance, gluing, sealing, shrinkage, clamping, screw thread or other fixing techniques known to a skilled person. In the wall 16, an incision 18 has been made with the first edge 12 which is designed as a cutting edge and/or provided with a sharp point 19 so that the incision 18 can be provided using the apparatus 1 in a leakproof manner. Incidentally, an incision may also first be made with another means such as a scalpel.

The point 19 or the longitudinal edge 12 of the flange part 7 is, as shown in Fig. 3B, pressed through the wall 16, such that the first edge 12 is brought under the wall 16 in the blood vessel. Then, as shown in Fig. 3C, the apparatus 1 is rotated about the longitudinal axis 4, in a direction Q where the first edge 12 is rotationally leading. Thus, the flange part 7 as a whole is forced under the wall 16 in the blood vessel 2, while the coupling element 3 is held outside the blood vessel. Surprisingly, thus, in a relatively smooth movement, the coupling element is pressed into the incision 18, while the edges 20A, 20B of the incision 18 are slightly elastically deformed and are forced against the coupling element 3 all round, as shown in Fig. 3D. In this condition, the wall 16 of the blood vessel 2 fittingly and sealingly connects against the outside of the coupling element 3 and the hollow tubular coupling element 3 extends through the wall 16, thus forming a connection between the inner space 21 of the blood vessel 2 and the second end 6 of the coupling element 3.

As shown in Fig. 3E, on or over the coupling element 3, a bypass vessel 17 can be slid and fixed, in the exemplary embodiment shown with the aid of an optionally roughened, perforated or tanned clamping ring 22. The clamping ring 22 is preferably pressed against the wall 16 of the blood vessel, so that the wall is locked between the clamping ring 22 and the flange part 7, while the bypass vessel 17 may optionally be clamped against the coupling element by the clamping ring 22 to prevent axial displacement and any coming loose. The clamping ring 22 thus acts as a stop element. Optionally, the second longitudinal edge 14 may be offset slightly upwards, for a still better locking. In the embodiment shown, the clamping ring 22 is provided with teeth 23 which may extend inwards and/or outwards. In the drawing, the teeth 23 are shown exaggeratedly large, for clarity. In practice, the teeth 23 may be designed to be smaller and less sharp or may, for instance, be omitted if a ring 22 with light drive fit can be used.

As can be seen in Fig. 2, the bottom side 23 of the flange part 7 is slightly convex or at least the longitudinal edge 10 thereof is rounded, in order to prevent obstruction in the blood vessel. In addition, this increases the safety because the wall 16 will not be damaged thereby. Further, the top side 24 of the first part 13 is preferably rounded, so inserting and rotating the apparatus 1 is further simplified. In addition, this more adequately keeps the longitudinal edge 12 at a distance from the wall 16.

In Fig. 4, in cross-sectional side view, an apparatus 1 according to the invention is placed in a wall 16 of a blood vessel 2, with a bypass vessel 17 on the coupling element 3. What is clearly visible is that the wall 16 of the blood vessel 2 is locked between the clamping ring 22 and the flange part 7. Because this flange part 7 extends through a relatively large angleα, for instance more than 320 degrees, this forms a good abutment surface. Incidentally, the flange part may also extend through more than 360 degrees or have interruptions. With an included angle of more than 360 degrees, a part of the flange part will be kept outside the blood vessel, so that a part of the wall 16 is included between parts of the flange part. In such an embodiment, the function of the clamping ring 22 is taken over by a part of the flange part 7.

Fig. 5 schematically shows a bypass vessel 17 included between two body channels, for instance blood vessels 2, fixed therein by two apparatuses 1 according to the invention. The bypass vessel 17 may be connected with the coupling elements 3 in any suitable manner and forms a direct connection between the two body channels 2. Incidentally, of course, also, a part of a body channel may be bridged by a bypass vessel 17 with the aid of apparatuses 1 according to the invention. On the bypass vessel 17, a clamp 25 is schematically shown, which temporarily closes off the passage of the bypass vessel 17, so that flow is prevented. This is particularly important during placement, in particular when the bypass vessel 17 has already been connected to the coupling element. Such a temporary closure may, incidentally, also be provided in a different manner, for instance in or to the coupling element 3. An apparatus according to the invention is preferably manufactured from a minimum number of parts, for instance two, the coupling element with the flange part on the one hand and the clamping ring on the other hand, or only one, if the flange part also has a locking function, but may also be assembled from more parts. Preferably, a biocompatible material is used, such as metal or plastic, and/or a coating which protects and/or covers the material. For instance, a coating may be used which has coagulation-resistant properties, a coating which simplifies sliding of the flange part along a vein wall or, conversely, prevents this, an adhesive coating which adheres to the vessel wall, a medicinal coating which, for instance, prevents restenosis or a protective layer to prevent rejection phenomena. Such coatings are known per se.

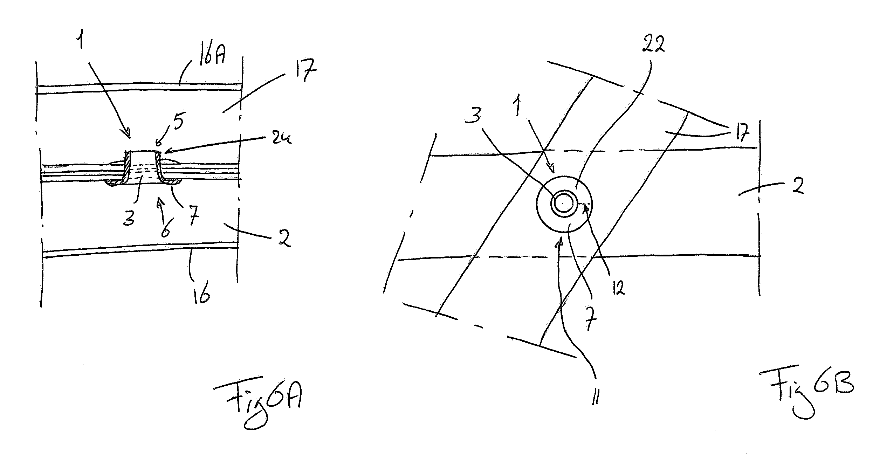

Figs. 6A and B show, in cross-sectional side view and partly transparently shown top plan view, an alternative embodiment of a connection between a blood vessel 2 and a bypass vessel 17, with the aid of an apparatus 1 according to the invention. With this embodiment, the longitudinal axes of the vessels 2, 17 are, at least at the location of the connection, approximately parallel or at least in approximately parallel planes and against each other, while the apparatus 1 has been inserted through the wall 16 of the blood vessel 2 in an above-described manner. To this end, in the embodiment shown, the bypass vessel 17 is laid against the outside of the wall 16 of the blood vessel 2, after which, from the inside of the bypass vessel 17, the apparatus 1 is run through the wall 16A of the bypass vessel, by piercing the edge 12 through this and then rotating the apparatus about the longitudinal axis 4 in above-described manner. Thus, viewed from the bypass vessel 17, the flange 7 is brought under the wall 16 of the vessel 2, while the shaft, i.e. the coupling element 3 is held inside the bypass vessel 17. Then, a clamping ring 22 is slid over the coupling element 3, against the inside of the wall 16A of the bypass vessel 17. Thus, a connection is obtained between the bypass vessel 17 and the vessel 2. In above-described manner, the bypass vessel can then be closed off temporarily and/or permanently at at least one end and/or be connected to another vessel 2. Incidentally, instead of a bypass vessel 17, in this manner, also another vessel 2 may be connected directly with a vessel 2, in a "side to side" connection. In such a connection, the length L of the coupling element 3 is preferably chosen so as to be minimal, preferably such that the transition from the clamping ring 22 to the longitudinal edge 24 of the first end 5 passes smoothly, so that flow of blood is not hindered or otherwise adversely affected, while the risk of deposition of body substances is reduced. Also, to this end, the clamping ring could, for instance, engage over the longitudinal edge 24 or even in the coupling element, for instance in a somewhat widened portion thereof.

The invention is by no means limited to the embodiments shown and described. Many variations thereof are possible within the framework of the invention set forth in the claims. Thus, the coupling element may have or still assume a different shape, for instance not round but somewhat oval or angular in cross section, and it may, for instance, have a cross section varying in the longitudinal direction, for instance in that the diameter increases at the location of the transition from the coupling element to the body channel to make it flow as naturally as possible. The coupling element is preferably relatively stiff, such that it cannot be squeezed shut by the vessel wall. It may have a longitudinal direction which includes a different angle with the flange part than shown, for instance with an obtuse or acute angle, so that, for instance, an Y-shaped connection can be obtained, which may be flow-technically advantageous, while the coupling element may also be designed so as to be at least partly bent or angular. The coupling element may have a closed wall but may also be wholly or partly provided with openings, for instance by manufacturing it from a gauze structure, similar to stents known per se, with a sufficient stiffness against radial compression. Adhesion to the bypass vessel can thus be simplified while the coupling element can be somewhat flexible so that it can bend in the longitudinal direction. In order to adequately clamp the edge of the receiving vessel, the flange part may be more than 360 degrees. Additionally, glue such as cyanoacrylate may also be used to reinforce the connection. For the manufacture, many techniques known per se may be used, optionally in combination, such as removing or non-removing deformation techniques, etching techniques and the like, while suitable surface treatments may be used, such as electropolishing operations, and using materials such as SMA and plastics or applying body-friendly coatings. These and similar variations are understood to fall within the invention and to be disclosed herein, both by themselves and in combination.

## Claims

1. An apparatus for bypass surgery on body channels such as blood vessels, comprising a body with at least a coupling element for coupling to a first body channel and a flange part with at least one cut, wherein at least a first part of the flange part which adjoins the cut is at least partly bent and/or displaced with respect to a part of the flange part on an opposite part of the cut.

2. An apparatus according to claim 1, wherein the coupling element extends from the flange part.

3. An apparatus according to claim 1 or 2, wherein the flange part extends through more than 320 degrees, in particular includes an angle between 320 and 360 degrees.

4. An apparatus according to any one of the preceding claims, wherein the flange part is slightly convex at least on a side remote from the coupling element.

5. An apparatus according to any one of the preceding claims, wherein said opposite part of the flange part is slightly bent in a direction away from the first part.

6. An apparatus according to any one of the preceding claims, wherein the coupling element is substantially tubular.

7. An apparatus according to any one of the preceding claims, wherein further a clamping device is provided for fixing a vessel on and/or to the coupling element.

8. An apparatus according to claim 7, wherein the clamping device and/or the coupling element and/or the flange part are provided with at least one stop element for preventing rotation of the clamping element relative to the flange part and/or the coupling element.

9. An apparatus according to any one of the preceding claims, wherein the flange part is substantially circular.

10. An apparatus according to any one of the preceding claims, wherein the first part has a cutting edge, in particular a cutting edge extending approximately radially.

11. An apparatus according to any one of the preceding claims, wherein the flange part is substantially ring-shaped with a central opening with an inner longitudinal edge with which the coupling element is connected, wherein the flange part between the inner longitudinal edge and an outer longitudinal edge has a radial dimension which is between 0.1 and 3 times a diameter of the central opening, more in particular between 0.5 and 1.5 times.

12. An apparatus according to claim 11, wherein the central opening has a diameter which is geared to the diameter of the vessel or body channel, for human blood vessels usually between approx. 0.5 and 5 mm.

13. An apparatus according to claim 11 or 12, wherein the coupling element extends in a tubular manner from the central opening and has a longitudinal direction, wherein, in said longitudinal direction, the length of the tubular coupling element has a length which is approximately equal to the diameter of said flange part.

14. An apparatus according to any one of the preceding claims, wherein at least the flange part is coated.

15. A set of an apparatus according to any one of the preceding claims and at least one natural or artificial blood vessel.

16. A set according to claim 15, wherein the blood vessel is couplable with the apparatus.

17. A set according to claim 15, wherein the blood vessel is fixedly connected with the apparatus.

18. A set according to any one of claims 15-17, wherein closing means are provided.

19. A method for use in interconnecting at least two parts of a body channel or two body channels such as blood vessels with the aid of an apparatus with at least a coupling element and a flange part with at least one cut, wherein at least a first part of the flange part which adjoins the cut is at least partly bent and/or displaced with respect to a part of the flange part on an opposite part of the cut, wherein, in a wall of one of the body channels, a cut is made, after which the said first part is introduced into the body channel through said cut and the apparatus is then rotated, such that the flange part is introduced into the body channel through said cut and the coupling element is kept at least partly outside the body channel.

20. A method according to claim 19, wherein the coupling element is tubular and is in open communication with a central opening in the flange part, wherein the apparatus is rotated such that said tubular coupling element runs through said cut and forms a connection between said body channel and an end of the tubular coupling element remote from the flange part.

21. A method according to claim 19 or 20, wherein a second body channel such as a natural or artificial blood vessel has been or is fixed on or to the coupling element.

22. A method according to any one of claims 19-21, wherein the coupling element or a further body channel connected thereto is closed off during placement of the apparatus.

23. A method for bypass surgery, wherein, with the aid of a method according to any one of claims 19-21, in two body channels to be interconnected such as blood vessels, a said apparatus is provided, wherein the coupling elements of said apparatuses are interconnected by a further body channel, which further body channel is an autograft, allograft or xenograft or an artificial body channel.
